# EUROPEAN PATENT SPECIFICATION

(11) **EP 1 590 020 B1**
(45) Date of publication and mention of the grant of the patent: **18.09.2013**
(21) Application number: 04702011.0
(22) Date of filing: 14.01.2004
(51) Int. Cl.: A61M 1/16, A61M 1/36

(54) **A SUPPORT DEVICE FOR CONTAINERS IN EXTRACORPOREAL BLOOD TREATMENT MACHINES**
STÜTZVORRICHTUNG FÜR BEHÄLTER IN MASCHINEN ZUR EXTRAKORPORALEN BLUTBEHANDLUNG
DISPOSITIF DE SUPPORT POUR RÉCIPIENTS DISPOSÉS DANS DES MACHINES DE TRAITEMENT SANGUIN EXTRACORPOREL

(30) Priority: 07.02.2003 IT MI20030216
(43) Date of publication of application: 02.11.2005
(73) Proprietor: Gambro Lundia AB, 22 643 Lund (SE)
(72) Inventor: MEZIERE, Cyril, F-69500 Bron (FR); BARALDI, Vincenzo, I-46026 Quistello (IT); TONELLI, Claudio, I-41100 Modena (IT); ZACCARELLI, Massimo, I-41038 San Felice sul Panaro (IT); CHEVALLET, Jacques, F-69360 Sérezin du Rhone (FR); FRESSINET, Jean Louis, F-69700 Saint Jean de Touslas (FR); AUDOUARD, Yves, F-69780 Moins (FR)
(74) Representative: Ponzellini, Gianmarco
(86) International application number: PCT/IB2004/000061
(87) International publication number: WO 2004/069312

(56) References cited:
- EP-A- 0 829 265
- US-A- 4 961 557
- US-A- 5 722 947
- US-B1- 6 390 311

## Description

### Background of the Invention.

The invention relates to a support device for containers, for extracorporeal blood treatment machines, or for renal failure treatment machines.

In more detail, the support device is destined to hold in position a predetermined number of bags containing the appropriate liquids destined for the various therapies which the patient will undergo.

As is known, the market already offers various machines for extracorporeal blood treatment, or for treatment of renal insufficiency, which machines are provided with respective support devices associated to the machine.

A first type of these support devices is constituted by arms, for example metal arms, which are engaged to the structure, directly constrained to the machine at an upper portion of the machine and provided at ends of the arms with one or more hooks which the bags containing the treatment liquids are attached to.

Another type of intensive therapy machine has these support devices located at a lower portion of the machine, so that the bags are attached in a position which is below the body of the machine.

Obviously, the second above type of machine has an improved stability with regard to the first, especially concerning jogs and sharp impacts in general to the machine in use, as the whole device's centre of gravity is kept as close as possible to the ground.

Though the prior art contains various bag support devices for machines destined for renal failure treatment or extracorporeal blood treatment, these devices have proved to be susceptible to improvement of various natures.

First of all, it is to be noted that the machines provided with support devices located above the machines themselves can lead not only to the above-mentioned problems connected with unexpected impacts and displacements, but also create problems related to the sometimes laborious and problematic operations of machine loading, i.e. the necessary lifting of a plurality of bags into the high position, and the need to make sure they are correctly engaged to the supports. On the other hand, machines with the support devices located in the lower portion of the machine body require, for reasons of stability, that the bags be positioned as close as possible to the vertical axis of the machine in order not to laterally displace the machine centre of gravity.

The above requirement leads to the need to position the containers below the machine body, in a zone which is difficult to access both visually and manually.

It is also known from US 6390311 a rack for medical bags, wherein the bags can be supported by telescopic arms. Moreover from document EP 829265 it is known a blood treatment machine having liquid bags positioned between the base and the body of the machine.

### Summary of the Invention.

In this situation, the main aim of the present invention is to resolve the above-described drawback in the prior art. Hence, a blood treatment machine as defined in claim 1 is provided.

A first aim of the invention is to maintain optimum stability of the machine in the face of impacts and displacements, both when the machine is not loaded with bags containing liquids and after the bags have been loaded.

A further aim of the invention is to provide a support device which enables easy bag-positioning operations, therefore ensuring, when the machine is being loaded, a simpler visual and manual access thereto.

Finally, a further aim of the invention is to limit the machine weight and dimensions as much as possible.

These and other aims will better emerge in the description that follows, of a support device for bags, for extracorporeal blood treatment machines or for renal failure treatment machines, according to what is set out in the appended claims.

### Brief Description of the Drawings.

Further characteristics and advantages of the invention will better emerge from the following detailed description of a specific embodiment, here described by way of nonlimiting example with reference to the figures of the drawings, in which:
- figure 1 is a side view of a support device according to the invention, in a minimum extension configuration;
- figure 2 is the device of figure 1, in a maximum extraction configuration;
- figure 3 is a side view of the device of figure 1;
- figures 4 and 5 are views from above of the device of figures 1 and 2;
- figure 6 is a schematic and perspective view of a machine for intensive therapy provided with the support device of figure 1;
- figure 7 is the machine of figure 6 loaded with a plurality of containers.

### Detailed Description.

With reference to the figures of the drawings, 1 denotes in its entirety a support device for containers or bags for machines for extracorporeal blood treatment or for renal insufficiency treatment.

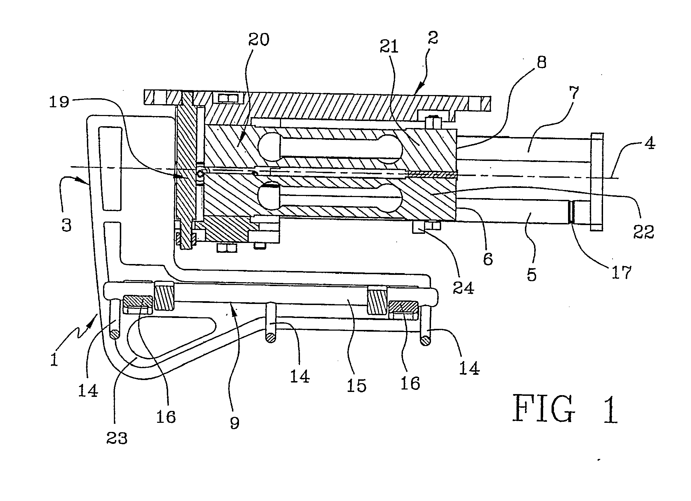

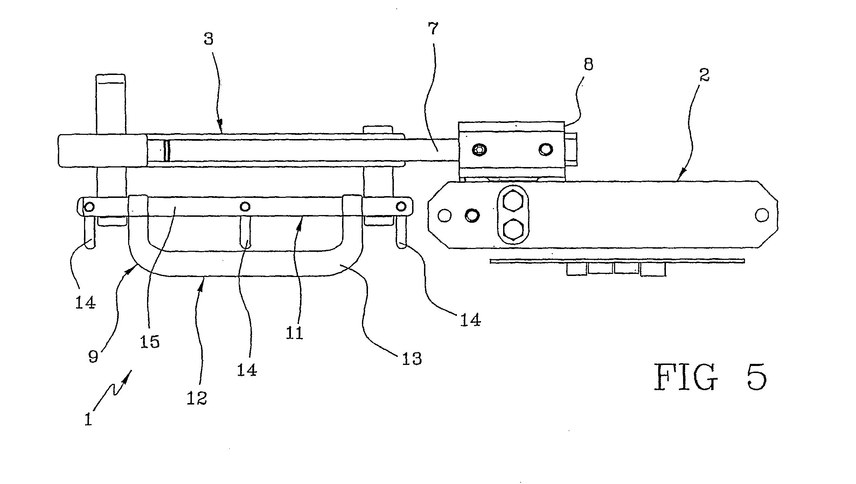

As can be observed in figures 1 to 5, the bag support device 1 comprises a base body 2, which base body 2 is generally (though not necessarily) constrained rigidly to a lower zone of a machine 100.

Obviously the device can be mounted in other parts of the machine, even above the machine body.

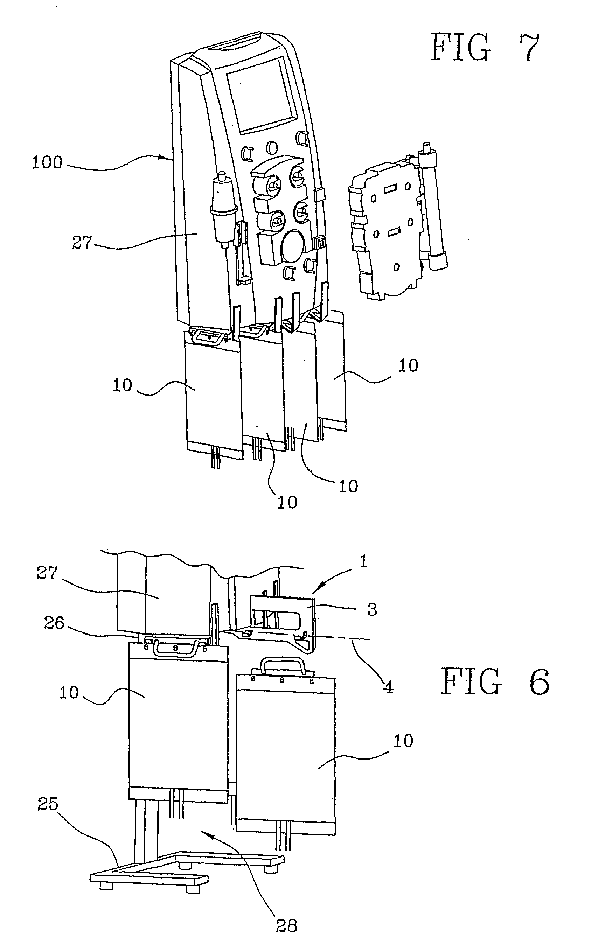

Generally speaking, however, the base body 2 is located below the body of the machine to enable a subsequent housing of the various bags 10 destined for use in the blood treatment operations or renal failure treatment operations in a free space afforded below the machine to which the support device is associated (see in particular figure 7, in which however the base body 2 is not visible).

In other words the machine affords a housing space 28 between the machine body 27, an upright structure 26 extending away from a floor base 25 and the same base 25.

The support device will be housed in the housing space 28 engaged to the machine body 27 and the upright structure 26.

This particular configuration means that the axis of the centre of gravity of the whole machine is maintained within the structure of the rest base when the machine is loaded with bags or when it is free of bags, whether the device is closed in the retracted position or open in the extracted position.

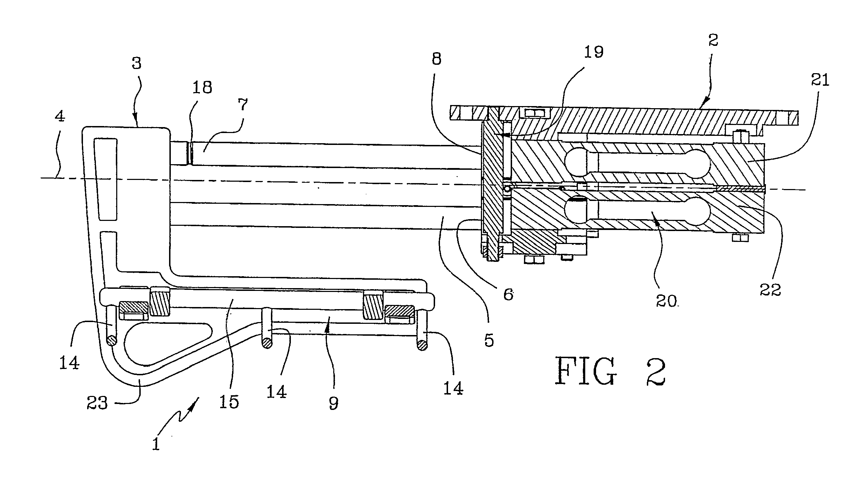

As well as the base body 2, the device also comprises a support element 3 associated to the base body 2, which support element 3 can be moved with respect to the base body 2 between at least an operative loading position (figures 2, 5 and 6) and an operative work position (figures 1, 4 and 7).

In other words, the support element 3 is slidingly mobile between the operative loading position, corresponding to an essentially maximum extraction position of the support element 3 from the base body 2, and the operative work position, corresponding to an essentially minimum extraction position of the support element 3 in relation to the base body 2.

In the illustrated embodiment, the support element 3 moves between the above-cited positions along a movement direction 4 lying in an essentially horizontal plane.

The support device for containers passes from one to the other position by translating movements.

A possible further embodiment could be inserting a hinge between the base body 2 and the support element 3 so that the displacement between the operative loading position and the operative work position could follow a rotary displacement or a combination of rotary and translating movements.

From a structural point of view the support element 3 exhibits at least one and, in the illustrated embodiment, two elongate arms 5, 7 which are slidable in respective guides 6, 8 of the base body 2, defining a telescopic structure.

Also, should a longer extraction run be necessary, it would be possible to include a telescopic structure having more than one telescopic guide, for example, one telescoping guide into another.

The support element 3 is provided with suitable means 9 for supporting a container 10.

The means 9 for supporting can be constituted by at least one body 11 which is removably constrainable to the support element 3.

The body 11 will be equipped with at least one support hook 14, and usually with at least two and specifically three support hooks 14, destined to receive respective containers 10.

The body 11 will also be provided with an organ for manual transport 12, for example a handle 13.

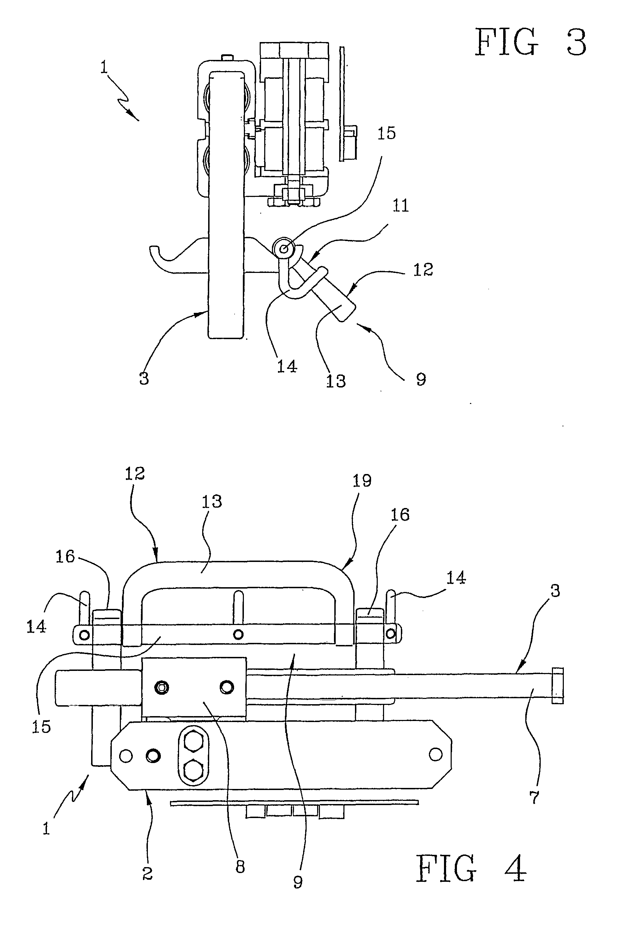

As the body 11 is removably constrainable to the support element 3, it can be separated there-from, a bag can be engaged on the respective hooks 14 and by means of the handle 13 the bag can be easily constrained to the support device by resting a rod 15 of the body 11 on special supports 16 exhibited by the support element and clearly visible in figures 2 and 3.

The support element 3 is further equipped with a manoeuvring handle 23 for enabling manual displacement between the operative work position and the operative loading position, and vice versa.

Obviously, instead of or together with the manoeuvring handle 23, an automatic movement system could be installed, in which the support element 3 could be commanded to displace between the various operative positions.

As can be observed in the figures, the support element 3 is provided with at least one mechanical endrun stop 17 for the operative loading position.

The endrun stop 17 can be a groove, for example located on the elongate arm 5 (see especially figure 1).

The support element 3 will also have at least one further endrun stop 18 for the operative work position.

In this case too, the mechanical endrun stop 18 can be a groove, for example, located on the elongate arm 7 (see especially figure 2).

The support device for bags further comprises at least one position sensor 19 associated to the base body 2 and able to recognise at least the arrival of the support element 3 in the operative work position.

The sensor will, usually, be a Hall sensor of known type and not further described herein.

Obviously, with the position sensor 19 it will be possible, should the need arise, to detect any position of the support element 3 relative to the base body 2.

The base body 2 is provided with weight sensors 20 for calculating the weight of a container 10 associated to the support device.

In particular, the sensors 20, for determining the weight, comprise at least one balance 21, which will send a signal proportional to the detected weight of the bag to a control unit. A further, controlling, balance 22 could be included to supply a further signal proportional to the weight of the bag, in effect controlling the reading of the other balance 21.

The device further comprises stop means 24 for selectively blocking the relative position of the support element 3 with respect to the base body 2 at least in the operative loading position and/or in the operative work position.

The stop means 24 can be constituted by a simple manually-activated or automatic pawl which cooperates with the elongate arms 5, 7 to block the arms in the desired relative positions.

Alternatively an actuator organ coordinated by a CPU can be included, to block at least one of the elongate arms 5, 7 on reaching a desired position.

The stop means 24 are normally active in blocking the support element in a retracted position, so as to prevent an undesired extraction of the arms 5, 7 when the machine is moved.

The stop means 24 are controlled, for example by an analog or digital control device, to enable contemporary extraction of a predetermined number of arms and bags. For example, if the predetermined number is one, the stop means 24 enable extraction of a single support at a time, automatically blocking the other supports in the retracted position. When the extracted support is returned to the retracted position the stop means 24 enable another or the same support to be extracted once more.

When the machine is unloaded, and loading of containers is about to begin, the support element is extracted, moving from the minimum extraction position from the base body 2 into the operative loading position (i.e. the maximum extraction position).

At this point the means 9 for supporting a container are brought into use, which are removed from the support element so that a bag can be engaged on the relative hooks.

Using the handle 13 the bag is positioned on the support element in a correct position and then the support element 3 is brought back into the operative work position.

The device may be designed so that the loading of a container 10 can be carried out exclusively in the operative loading position of the support element 3, in order to avoid the possibility of incorrect assembly operations.

The signal coming from the balance 21 will be sent and read as correct only when the container and the support element 3 are in the operative work position.

The invention offers important advantages.

Firstly, the use of a support device having a telescopic structure affords a very easy and functional loading operation of the bags for dialysis treatment and/or blood treatment on the patient.

In particular, as the support element 3 can be extracted, visual and manual access becomes extremely easy, and being in the lower position access for the operator is extremely comfortable.

Once the support arm is brought into the operative work position the added weight due to the presence of the bags is located as closely as possible to the ground and to machine centre of gravity.

Thus stability in the face of jogs, impacts and sharp movements is optimised; and the space taken up by the whole machine is diminished.

All of the various activities of the machine, controlled by a control unit, are started up only when the bag is correctly positioned below the machine, preventing erroneous activation on the part of the operator or activation of the machine when working conditions are not optimal.

## Claims

1. A machine for extracorporeal blood treatment or for treatment of renal failure, comprising:
- a floor base (25);
- an upright structure (26) developing away from the floor base (25);
- a machine body (27) borne superiorly by the upright structure (26) and defining a housing space (28) together with the floor base (25); and
- support devices (1) for containers of liquids constrained to the machine body (27) or to the upright structure (26) at a position corresponding to the housing space (28), each support device comprising:
- a base body (2); and
- a support element (3) associated to the base body (2),
**characterized in that** the support element (3) is displaceable with respect to the base body (2) between at least one operative loading position, corresponding to an essentially maximum extraction position of the support element (3) from the base body (2), and an operative work condition, corresponding to an essentially minimum extraction position of the support element (3) from the base body (2),
and **in that** stop means (24) are provided for selectively blocking a relative position of the support element (3) with respect to the base body (2), said stop means (24) being normally active for blocking the support element (3) in the operative work position thereof, the stop means (24) enabling a contemporary extraction only of a predetermined number of support elements (3).

2. The machine of claim 1, **characterized in that** the support element (3) is slidable between the operative loading position and the operative work position along a movement direction (4).

3. The machine of claim 2, **characterized in that** the movement direction (4) lies in an essentially horizontal plane when the support device is operating.

4. The machine of claim 1, **characterized in that** the support element (3) is mobile between the operative loading position and the operative work position by means of at least a translating or rotary displacement.

5. The machine of claim 2, **characterized in that** the support element (3) comprises at least one elongate arm (5) which is slidable in a guide (6) of the base body (2) in order to displace between the operative loading position and the operative work position.

6. The machine of claim 5, **characterized in that** the support element (3) comprises two elongate arms (5, 7) which are slidable in guides (6, 8) of the base body (2) in order to displace between the operative loading position and the operative work position.

7. The machine of any one of the preceding claims, **characterized in that** the support element (3) comprises means for supporting (9) a container (10).

8. The machine of claim 7, **characterized in that** the means for supporting (9) comprise at least one body (11) which is removably constrainable to the support element (3) for supporting the said container (10).

9. The machine of claim 8, **characterized in that** the body (11) which is constrainable to the support element (3) exhibits a manual transport organ (12) and at least one support hook (14) for the said container (10).

10. The machine of claim 9, **characterized in that** the manual transport organ (12) is a handle (13).

11. The machine of claim 9, **characterized in that** the body (11) comprises at least two support hooks (14) for receiving the said container (10).

12. The machine of claim 9, **characterized in that** the body (11) constrainable to the support element (3) comprises a rod (15) which bears the manual transport organ (12) and the said at least one support hook (14), the support element (3) exhibiting supports (16) for receiving and engaging the rod (15).

13. The machine of any one of the preceding claims, **characterized in that** the support element (3) is provided with at least one mechanical endrun stop (17) for the operative loading position.

14. The machine of claim 13, **characterized in that** the mechanical endrun stop (17) is defined by a groove located on the said elongate arm (5).

15. The machine of claim 13 or 14, **characterized in that** the support element (3) is provided with at least one further mechanical endrun stop (18) for the operative work position.

16. The machine of claim 15, **characterized in that** the further mechanical endrun stop (18) is defined by a groove located on an elongate arm (7).

17. The machine of any one of the preceding claims, **characterized in that** it comprises at least one position sensor (19), associated to the base body (2), for detecting at least the operative work position of the support element (3).

18. The machine of claim 17, **characterized in that** the position sensor (19) is a Hall sensor.

19. The machine of any one of the preceding claims, **characterized in that** it comprises further sensors (20) for weighing a container (10) associated to the support device.

20. The machine of claim 19, **characterized in that** the sensors (20) for weighing comprise at least one measuring balance (21).

21. The machine of claim 20, **characterized in that** the sensors (20) for weighing further comprise a control balance (22).

22. The machine of any one of the preceding claims, **characterized in that** the support element (3) further comprises a manoeuvring handle (23) for enabling a manual displacement between the operative work position and the operative loading position, and vice versa.

23. The machine of claim 20, comprising a machine control unit, which is provided with a CPU which receives a signal proportional to a weight provided by the balance (21) for weighing; the said CPU being able to validate the said signal relating to the weight only when the support element (3) is in the operative work position.

24. The machine of any one of the preceding claims, **characterized in that** the base body (2) is associated to the machine at a lower portion of the machine.

25. Machine according to claim 1, further comprising an analog or digital control device controlling the stop means to enable the contemporary extraction of a predetermined number of support elements.

26. Machine according to claim 25, wherein, if the predetermined number is one, the stop means (24) enable extraction of a single support at a time, automatically blocking the other supports in the retracted position corresponding to the operative work condition.

## Patentansprüche

1. Maschine zur extrakorporalen Blutbehandlung oder zur Behandlung von Nierenversagen, umfassend:
- eine Bodenplatte (25);
- eine aufrechte Struktur (26), die sich weg von der Bodenplatte (25) entwickelt;
- einen Maschinenkörper (27), der von der aufrechten Struktur (26) auf deren Oberseite getragen wird und zusammen mit der Bodenplatte (25) einen Gehäuseraum (28) definiert; und
- Trägervorrichtungen (1) für Flüssigkeitsbehälter, die mit dem Maschinenkörper (27) oder mit der aufrechten Struktur (26) an einer Stelle, die dem Gehäuseraum (28) entspricht, befestigt sind, jede Trägervorrichtung umfassend:
- einen Grundkörper (2); und
- ein Trägerelement (3), das dem Grundkörper (2) zugeordnet ist,
**dadurch gekennzeichnet, dass** das Trägerelement (3) gegenüber dem Grundkörper (2) zwischen mindestens einer Betriebsladestellung, die einer im Wesentlichen maximalen Ausfahrstellung des Trägerelements (3) aus dem Grundkörper (2) entspricht, und einer Betriebsarbeitsstellung, die einer im Wesentlichen minimalen Ausfahrstellung des Trägerelements (3) aus dem Grundkörper (2) entspricht, verstellbar ist,
und dass Arretierungsmittel (24) zur selektiven Verriegelung einer relativen Stellung des Trägerelements (3) gegenüber dem Grundkörper (2) vorgesehen sind, wobei die Arretierungsmittel (24) zur Verriegelung des Trägerelements (3) in dessen Betriebsarbeitsstellung in der Regel aktiv sind, wobei die Arretierungsmittel (24) ein gleichzeitiges Ausfahren von nur einer vorgegebenen Anzahl von Trägerelementen (3) ermöglichen.

2. Maschine nach Anspruch 1, **dadurch gekennzeichnet, dass** das Trägerelement (3) zwischen der Betriebsladestellung und der Betriebsarbeitsstellung entlang einer Bewegungsrichtung (4) verschiebbar ist.

3. Maschine nach Anspruch 2, **dadurch gekennzeichnet, dass** die Bewegungsrichtung (4) in einer im Wesentlichen horizontalen Ebene liegt, wenn die Trägervorrichtung in Betrieb ist.

4. Maschine nach Anspruch 1, **dadurch gekennzeichnet, dass** das Trägerelement (3) zwischen der Betriebsladestellung und der Betriebsarbeitsstellung durch mindestens eine Translations- oder Rotationsverstellung beweglich ist.

5. Maschine nach Anspruch 2, **dadurch gekennzeichnet, dass** das Trägerelement (3) mindestens einen länglichen Arm (5) umfasst, der in einer Führung (6) des Grundkörpers (2) verschiebbar ist, um zwischen der Betriebsladestellung und der Betriebsarbeitsstellung zu verstellt zu werden.

6. Maschine nach Anspruch 5, **dadurch gekennzeichnet, dass** das Trägerelement (3) zwei länglichen Arme (5, 7) umfasst, die in Führungen (6, 8) des Grundkörpers (2) verschiebbar sind, um zwischen der Betriebsladestellung und der Betriebsarbeitsstellung zu verstellt zu werden.

7. Maschine nach einem der vorhergehenden Ansprüche, **dadurch gekennzeichnet, dass** das Trägerelement (3) Trägermittel (9) für einen Behälter (10) umfasst.

8. Maschine nach Anspruch 7, **dadurch gekennzeichnet, dass** die Trägermittel (9) mindestens einen Körper (11) umfassen, der am Trägerelement (3) zum Tragen des Behälters (10) lösbar befestigt werden kann.

9. Maschine nach Anspruch 8, **dadurch gekennzeichnet, dass** der Körper (11), der am Trägerelement (3) befestigt werden kann, ein Handbeförderungsglied (12) und mindestens einen Trägerhaken (14) für den Behälter (10) aufweist.

10. Maschine nach Anspruch 9, **dadurch gekennzeichnet, dass** das Handbeförderungsglied (12) ein Griff (13) ist.

11. Maschine nach Anspruch 9, **dadurch gekennzeichnet, dass** der Körper (11) mindestens zwei Trägerhaken (14) zur Aufnahme des Behälters (10) umfasst.

12. Maschine nach Anspruch 9, **dadurch gekennzeichnet, dass** der Körper (11), der am Trägerelement (3) befestigt werden kann, eine Stange (15) umfasst, die das Handbeförderungsglied (12) und den mindestens einen Trägerhaken (14) trägt, wobei das Trägerelement (3) Träger (16) zur Aufnahme und zum Eingriff der Stange (15) aufweist.

13. Maschine nach einem der vorhergehenden Ansprüche, **dadurch gekennzeichnet, dass** das Trägerelement (3) mit mindestens einem mechanischen Endanschlag (17) für die Betriebsladestellung ausgestattet ist.

14. Maschine nach Anspruch 13, **dadurch gekennzeichnet, dass** der mechanische Endanschlag (17) von einer am länglichen Arm (5) liegenden Nut definiert ist.

15. Maschine nach Anspruch 13 oder 14, **dadurch gekennzeichnet, dass** das Trägerelement (3) mit mindestens einem weiteren mechanischen Endanschlag (18) für die Betriebsarbeitsstellung ausgestattet ist.

16. Maschine nach Anspruch 15, **dadurch gekennzeichnet, dass** der weitere mechanische Endanschlag (18) von einer an einem länglichen Arm (7) liegenden Nut definiert ist.

17. Maschine nach einem der vorhergehenden Ansprüche, **dadurch gekennzeichnet, dass** sie mindestens einen Positionssensor (19) umfasst, der dem Grundkörper (2) zugeordnet ist, zur Erfassung mindestens der Betriebsarbeitsstellung des Trägerelements (3).

18. Maschine nach Anspruch 17, **dadurch gekennzeichnet, dass** der Positionssensor (19) ein Hall-Sensor ist.

19. Maschine nach einem der vorhergehenden Ansprüche, **dadurch gekennzeichnet, dass** sie weitere Sensoren (20) zum Wiegen eines der Trägervorrichtung zugeordneten Behälters (10) umfasst.

20. Maschine nach Anspruch 19, **dadurch gekennzeichnet, dass** die Sensoren (20) zum Wiegen mindestens eine Messwaage (21) umfassen.

21. Maschine nach Anspruch 20, **dadurch gekennzeichnet, dass** die Sensoren (20) zum Wiegen eine Kontrollwaage (22) weiter umfassen.

22. Maschine nach einem der vorhergehenden Ansprüche, **dadurch gekennzeichnet, dass** das Trägerelement (3) weiter einen Steuergriff (23) zum Ermöglichen eines manuellen Verstellens zwischen der Betriebsarbeitsstellung und der Betriebsladestellung, und umgekehrt, umfasst.

23. Maschine nach Anspruch 20, umfassend eine Maschinensteuereinheit, die mit einer Zentraleinheit ausgestattet ist, die ein Signal proportional zu einem von der Waage (21) gelieferten Gewicht empfängt; wobei die Zentraleinheit das Gewichtssignal erst validieren kann, wenn das Trägerelement (3) in der Betriebsarbeitsstellung ist.

24. Maschine nach einem der vorhergehenden Ansprüche, **dadurch gekennzeichnet, dass** der Grundkörper (2) mit der Maschine an einem unteren Teil davon verbunden ist.

25. Maschine nach Anspruch 1, weiter umfassend eine Analog- oder Digitalsteuervorrichtung, die die Arretierungsmittel steuert, um das gleichzeitige Ausfahren einer vorgegebenen Anzahl von Trägerelementen zu ermöglichen.

26. Maschine nach Anspruch 25, wobei, wenn die vorgegebene Anzahl eins ist, die Arretierungsmittel (24) das Ausfahren jeweils eines einzigen Trägerelements nach dem anderen ermöglichen, wobei die anderen Träger in der eingefahrenen Stellung entsprechend der Betriebsarbeitsstellung automatisch verriegeln werden.

## Revendications

1. Machine pour le traitement extracorporel du sang ou pour le traitement de l'insuffisance rénale, comprenant:
- une base (25);
- une structure verticale (26) qui se développe en s'éloignant de la base (25);
- un corps de machine (27) porté supérieurement par la structure verticale (26) et définissant un espace de logement (28) avec la base (25); et
- des dispositifs de support (1) pour récipients de liquides fixés au corps de machine (27) ou à la structure verticale (26) dans une position correspondant à l'espace de logement (8), chaque dispositif de support comprenant:
- un corps de base (2); et
- un élément de support (3) associé au corps de base (2),
**caractérisée en ce que** l'élément de support (3) est déplaçable par rapport au corps de base (2) entre au moins une position de chargement opérationnelle, correspondant à une position d'extraction essentiellement maximum de l'élément de support (3) du corps de base (2), et une position de travail opérationnelle, correspondant à une position d'extraction essentiellement minimum de l'élément de support (3) du corps de base (2),
et **en ce que** des moyens d'arrêt (24) sont prévus pour bloquer sélectivement une position relative de l'élément de support (3) par rapport au corps de base (2), lesdits moyens d'arrêt (24) étant normalement actifs pour bloquer l'élément de support (3) dans sa position de travail opérationnelle, les moyens d'arrêt (24) permettant une extraction simultanée seulement d'un nombre préétabli d'éléments de support (3).

2. Machine selon la revendication 1, **caractérisée en ce que** l'élément de support (3) est coulissant entre la position de chargement opérationnelle et la position de travail opérationnelle le long d'une direction de mouvement (4).

3. Machine selon la revendication 2, **caractérisée en ce que** la direction de mouvement (4) se trouve dans un plan essentiellement horizontal lorsque le dispositif de support est en fonction.

4. Machine selon la revendication 1, **caractérisée en ce que** l'élément de support (3) est mobile entre la position de chargement opérationnelle et la position de travail opérationnelle au travers d'au moins une déplacement de translation ou de rotation.

5. Machine selon la revendication 2, **caractérisée en ce que** l'élément de support (3) comprend au moins un bras allongé (5) coulissant dans un guide (6) du corps de base (2) pour se déplacer entre la position de chargement opérationnelle et la position de travail opérationnelle.

6. Machine selon la revendication 5, **caractérisée en ce que** l'élément de support (3) comprend deux bras allongés (5, 7) coulissant dans des guides (6, 8) du corps de base (2) pour se déplacer entre la position de chargement opérationnelle et la position de travail opérationnelle.

7. Machine selon une quelconque des revendications précédentes, **caractérisée en ce que** l'élément de support (3) comprend des moyens de support (9) pour un récipient (10).

8. Machine selon la revendication 7, **caractérisée en ce que** les moyens de support (9) comprennent au moins un corps (11) pouvant être fixé de façon amovible à l'élément de support (3) pour supporter ledit récipient (10).

9. Machine selon la revendication 8, **caractérisée en ce que** le corps (11) pouvant être fixé à l'élément de support (3) présente un organe de transport manuel (12) et au moins un crochet de support (14) pour ledit récipient (10).

10. Machine selon la revendication 9, **caractérisée en ce que** l'organe de transport manuel (12) est une poignée (13).

11. Machine selon la revendication 9, **caractérisée en ce que** le corps (11) comprend au moins deux crochets de support (14) aptes à recevoir ledit récipient (10).

12. Machine selon la revendication 9, **caractérisée en ce que** le corps (11) pouvant être fixé à l'élément de support (3) comprend une tige (15) portant l'organe de transport manuel (12) et ledit au moins un crochet de support (14), l'élément de support (3) présentant des supports (16) aptes à recevoir et engager la tige (15).

13. Machine selon une quelconque des revendications précédentes, **caractérisée en ce que** l'élément de support (3) est muni d'au moins une butée mécanique (17) pour la position de chargement opérationnelle.

14. Machine selon la revendication 13, **caractérisée en ce que** la butée mécanique (17) est définie par une rainure placée sur ledit bras allongé (5).

15. Machine selon la revendication 13 ou 14, **caractérisée en ce que** l'élément de support (3) est muni d'au moins une autre butée mécanique (18) pour la position de travail opérationnelle.

16. Machine selon la revendication 15, **caractérisée en ce que** l'autre butée mécanique (18) est définie par une rainure placée sur un bras allongé (7).

17. Machine selon une quelconque des revendications précédentes, **caractérisée en ce que** elle comprend au moins un capteur de position (19) associé au corps de base (2) pour détecter au moins la position de travail opérationnelle de l'élément de support (3).

18. Machine selon la revendication 17, **caractérisée en ce que** le capteur de position (19) est un capteur à effet Hall.

19. Machine selon une quelconque des revendications précédentes, **caractérisée en ce que** elle comprend d'autres capteurs (20) aptes à peser un récipient (10) associé au dispositif de support.

20. Machine selon la revendication 19, **caractérisée en ce que** les capteurs de poids (20) comprennent au moins une balance de mesure (21).

21. Machine selon la revendication 20, **caractérisée en ce que** les capteurs de poids (20) comprennent en outre une balance de contrôle (22).

22. Machine selon une quelconque des revendications précédentes, **caractérisée en ce que** l'élément de support (3) comprend en outre une poignée de manoeuvre (23) apte à permettre un déplacement manuel entre la position de travail opérationnelle et la position de chargement opérationnelle et vice-versa.

23. Machine selon la revendication 20, comprenant une unité de commande de la machine munie d'une unité centrale recevant un signal proportionnel à un poids fourni par la balance (21) pour peser; ladite unité centrale étant à même de valider ledit signal de poids seulement lorsque l'élément de support (3) est dans la position de travail opérationnelle.

24. Machine selon une quelconque des revendications précédentes, **caractérisée en ce que** le corps de base (2) est associée à la machine dans une portion inférieure de la machine.

25. Machine selon la revendication 1, comprenant en outre un dispositif de commande analogue ou numérique commandant les moyens d'arrêt pour permettre l'extraction simultanée d'un nombre préétabli d'éléments de support.

26. Machine selon la revendication 25, où, si le nombre préétabli est un, les moyens d'arrêt (24) permettent l'extraction d'un seul support à la fois, bloquant ainsi automatiquement les autres supports dans la position rentrée correspondant à la position de travail opérationnelle.
